# EUROPEAN PATENT SPECIFICATION

(11) **EP 3 732 378 B1**
(45) Date of publication and mention of the grant of the patent: **27.11.2024**
(21) Application number: 18895917.5
(22) Date of filing: 28.12.2018
(51) Int. Cl.: F04D 13/06, F04D 29/047, F04D 29/22, F04D 29/24, F04D 29/18, F04D 29/041, F04D 29/66, A61M 60/205, A61M 60/824

(54) **PUMP**
PUMPE
POMPE

(30) Priority: 29.12.2017 US 201762612014 P
(43) Date of publication of application: 04.11.2020
(73) Proprietor: Saint-Gobain Performance Plastics Corporation, Solon, OH 44139 (US)
(72) Inventor: PAGLIARO, Anthony, P., Lansdale, PA 19446 (US); HARMAN, Jayden, David, Princeville, HI 96722 (US)
(74) Representative: Zimmermann & Partner Patentanwälte mbB
(86) International application number: PCT/US2018/067853
(87) International publication number: WO 2019/133807

(56) References cited:
- CN-A- 103 083 742
- US-A- 5 934 877
- US-A- 5 934 877
- US-A- 6 015 272
- US-A- 6 053 705
- US-A1- 2007 077 155
- US-A1- 2014 023 535
- US-A1- 2016 287 771
- US-B1- 6 702 552

## Description

### TECHNICAL FIELD

The present disclosure relates to pumps, and more particularly to in-line pumps.

### BACKGROUND ART

Pumps are generally used to transport fluid between two locations. In biopharmaceutical applications, for example, pumps may be used to move biopharmaceutical materials between various storage containers, mixers, testing equipment, and other areas. Conventional pumps include drive shafts and rotating impellers imparting damaging forces along shear-sensitive fluidic components. Conventional pumps can impart uneven fluid flow rates and uneven pulsations which can damage or disturb sensitive systems.

US 5 934 877 A discloses a rotor for use with a fluid flow generator or reactor, the rotor being intended to rotate about a central axis and having a surface which defines an arcuate fluid pathway for fluid flow about the central axis about which the rotor is able to rotate, wherein the surface has the configuration of a logarithmic curve substantially conforming to the mathematical progression known as the Golden Section or the Fibonacci Progression.

### BRIEF DESCRIPTION OF THE DRAWINGS

Embodiments are illustrated by way of example and are not limited in the accompanying figures.
FIG. 1 includes a cross-sectional schematic view of a system in accordance with an embodiment.
FIG. 2 includes an enlarged cross-sectional view of a fluid bearing formed in the system in accordance with an embodiment.
FIG. 3 includes a top view of an impeller in accordance with an embodiment.
FIG. 4 includes a schematic view of a system in accordance with an embodiment.
FIG. 5 includes a view of an exemplary housing in accordance with an embodiment.
FIG. 6 includes an exemplary pump with a semi-transparent housing and pump in accordance with an embodiment.
FIG. 6a includes multiple views of an exemplary impeller in accordance with an embodiment.
FIG. 6b includes a side view of an exemplary housing and impeller in accordance with an embodiment.
FIG. 6c includes a rotated side view of the exemplary housing and impeller of FIGs. 6a and 6b.
FIG. 6d includes a top view and a bottom view, respectively, of the exemplary housing and impeller of FIGs. 6a and 6b.
FIG. 6e includes a top exploded view of an exemplary housing and impeller.

Skilled artisans appreciate that elements in the figures are illustrated for simplicity and clarity and have not necessarily been drawn to scale. For example, the dimensions of some of the elements in the figures may be exaggerated relative to other elements to help to improve understanding of embodiments of the invention.

### DETAILED DESCRIPTION OF THE PREFERRED EMBODIMENT(S)

The following description in combination with the figures is provided to assist in understanding the teachings disclosed herein. The following discussion will focus on specific implementations and embodiments of the teachings. This focus is provided to assist in describing the teachings and should not be interpreted as a limitation on the scope of the invention, which is solely defined by the appended claims.

As used herein, the terms "comprises," "comprising," "includes," "including," "has," "having," or any other variation thereof, are intended to cover a non-exclusive inclusion. For example, a process, method, article, or apparatus that comprises a list of features is not necessarily limited only to those features but may include other features not expressly listed or inherent to such process, method, article, or apparatus. Further, unless expressly stated to the contrary, "or" refers to an inclusive-or and not to an exclusive-or. For example, a condition A or B is satisfied by any one of the following: A is true (or present) and B is false (or not present), A is false (or not present) and B is true (or present), and both A and B are true (or present).

The use of "a" or "an" is employed to describe elements and components described herein. This is done merely for convenience and to give a general sense of the scope of the invention. This description should be read to include one or at least one and the singular also includes the plural, or vice versa, unless it is clear that it is meant otherwise.

Unless otherwise defined, all technical and scientific terms used herein have the same meaning as commonly understood by one of ordinary skill in the art to which this invention belongs. The materials, methods, and examples are illustrative only and not intended to be limiting. To the extent not described herein, many details regarding specific materials and processing acts are conventional and may be found in textbooks and other sources within the fluid and pump arts.

A pump in accordance with the present invention is defined by claim 1 and includes, among other features, a housing defining an internal volume and an impeller disposed in the internal volume. The impeller includes a magnetic element adapted to magnetically couple with a drive element. The drive element, which can be externally located with respect to the housing, magnetically urges the impeller. At least a portion of the surface of the impeller conforms to a logarithmic spiral.

A single-use pump in accordance with one or more of the embodiments described but which is not claimed can generally include a housing defining an internal volume and an impeller disposed in the internal volume. At least a portion of the surface of the impeller can conform to a generally logarithmic spiral. The single-use pump is adapted to magnetically couple with a drive element. The drive element can be reusable between different pumping operations.

A fluid circulation system in accordance with one or more of the embodiments described herein includes first and second tubulars, and a pump according to the present invention disposed between the first and second tubulars.

In certain embodiments which are not claimed, a biopharmaceutical circulation system can include first and second tubulars coupled to first and second biopharmaceutical equipment, respectively. A pump disposed between the first and second tubulars can urge fluid from the first tubular to the second tubular. The pump can include a housing defining an internal volume and an impeller disposed in the internal volume. The impeller can include a magnetic element and at least a portion of the surface of the impeller can conform to a generally logarithmic spiral. A drive element can magnetically couple with the magnet element of the impeller and rotationally urge the impeller so as to create fluid movement within the system.

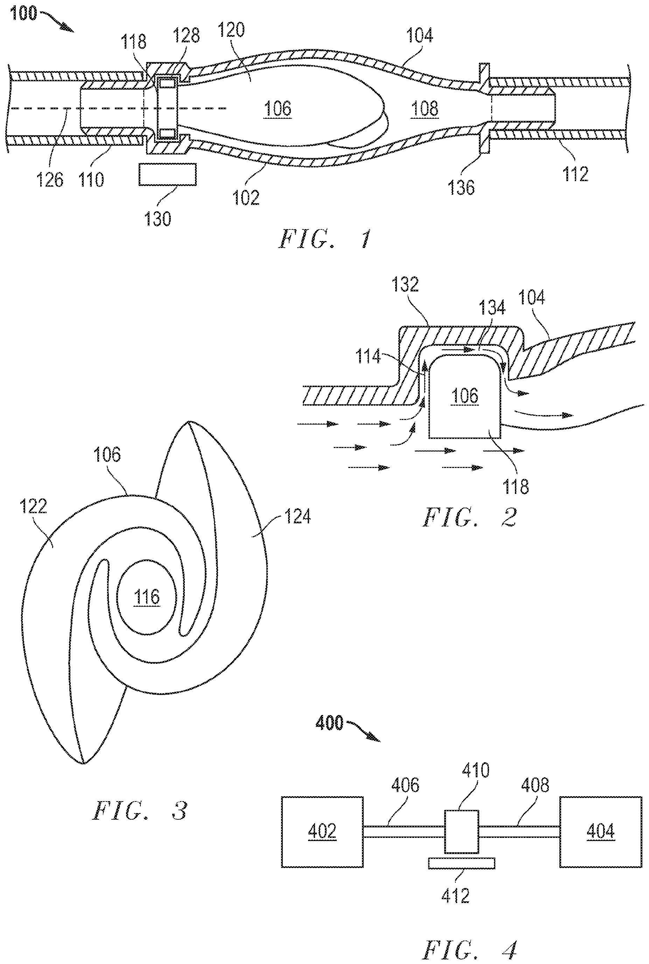

Referring to FIG. 1, a fluid system 100 can generally include a pump 102. The pump 102 includes a housing 104 and an impeller 106. As illustrated, the impeller 106 seats within an internal volume 108 of the housing 102. The internal volume 108 can define an internal volume between first and second tubulars 110 and 112. The first tubular 110 can be coupled with a first equipment, such as a biopharmaceutical or medical equipment, and the second tubular 112 can be coupled with a second equipment, such as a biopharmaceutical or medical equipment. The equipment can include a storage container, a mixer, a sampling device, a testing device, other suitable equipment used in manufacturing or development of biopharmaceutical materials, or any combination thereof.

In an embodiment, fluid flow through the system 100 is unidirectional. In a particular embodiment, fluid flow occurs in a direction generally from the first tubular 110 to the second tubular 112. In another embodiment, fluid flow through the system 100 is bidirectional. Rotation of the impeller 106 can generate axial fluid pressure, causing fluid movement through the system 100.

In an embodiment, the housing 104 includes at least two discrete components. The at least two discrete components can be detachably coupled together. In an embodiment, the impeller 106 can be installed within the internal volume 108 prior to connecting the at least two discrete components together.

The internal volume 108 can have a curved interior volume defined by a curved sidewall of the housing 104. Fluid flow through the system 100 occurs at least in part around an outer surface of the impeller 106. Referring to FIG. 2, the impeller 106 forms a fluid bearing with an inner surface of the housing 104. Fluid entering the pump 102 from the first tubular 110 passes into the housing 104 and moves through an opening 114 located between the impeller 106 and housing 104. As the impeller 106 begins rotating, fluid passing by the impeller 106 creates a fluid bearing which maintains the impeller 106 in a rotationally lubricated state. The fluid bearing can also permit self-centering of the impeller 106 relative to the housing 104. Fluid not directed through the fluid bearing between the housing 104 and impeller 106 passes through an aperture 116 (FIG. 3) in the impeller 106. As described below in greater detail, the fluid passing through the aperture is driven by vanes of the impeller 106.

In the illustrated embodiment, the impeller 106 includes a hub 118 and a blade 120 extending from the hub 118. The aperture 116 extends through the impeller 106, passing through the hub 118 and the blade 120. As fluid passed through the aperture 116, it is urged by the blade 120 in a direction generally parallel with an axis 126 of the impeller 106.

The blade 120 includes vanes 122 and 124 (FIG. 3) adapted to bias the fluid through the system 100. According to the invention, the blade 120 includes at least two vanes. In an embodiment, the blade 120 can include at least three vanes, or at least four vanes, or at least five vanes, or even at least ten vanes. In another embodiment, the blade 120 can include no greater than 1000 vanes, no greater than 500 vanes, or even no greater than 50 vanes. The vanes 122 and 124 can have a shape, when viewed along the axis 126 of the impeller 106, generally corresponding to a Fibonacci sequence, also known as a golden ratio. Viewed in cross section, the impeller can have a surface curvature generally conforming to the Fibonacci sequence pattern. Impellers with such pattern have been found to be particularly effective at providing even fluid flow and dynamic balance, while exhibiting non-destructive fluid behavior (i.e., not destroying any media, e.g., biological media, within the fluid) and avoiding clogs common among conventionally-shaped impellers. Any length, width, or shape of the blade is envisioned. In an embodiment, the blade 120 can have vanes 120 and 124 having a tulip shape.

When the impeller 106 rotates within the housing 104, it creates axial flow of the fluid in the internal volume 108 in a direction generally corresponding to the axis 126 of rotation. As illustrated in FIG. 1, and in accordance with an embodiment, fluid flow can occur in a left-to-right direction. In another embodiment, fluid flow can occur in a right-to-left direction. Fluid flow direction is a product of blade 120 design and rotational direction. Although illustrated in a 180 degree path with the fluid flow through the pump 102 as linear, in an alternative embodiment, the fluid flow may be divergent through one or more exit paths from greater than 0 degrees to less than 180 degrees on any axis.

At first, the impeller 106 generates fluid turbulence as the fluid is initially stationary. As the impeller 106 begins rotating and circulation begins to occur, the turbulence subsides and cavitation is minimized. In a particular embodiment, the pump 102 provides cavitation-free fluid flow. Cavitation-free fluid flow provides desired properties such as, for example, a reduction in noise levels, an increase in electrical efficiency, the elimination or reduction of pump damage during operation, the like, or any combination thereof. This is contrary to conventional impellers. Conventional impellers, for example, may have pump cavitation which creates bubbles and causes unpredictable shear forces on the pumped fluid and also undesirably increases system pressure. The impeller 106 is configured to allow biasing of higher volumetric flow rates without damaging or disturbing shear-sensitive media suspended within the fluid. Additionally, because the impeller 106 biases fluid through a central aperture 116 (FIG. 3), and not along an outer perimeter typical of conventional impellers, the speed of liquid flow is greatest in the center of the internal volume 108 where frictional resistance caused by sidewalls of the tubulars 110 and 112 is greatest. The lack of any traditional leading edge on the impeller 106 further reduces media degradation typically associated with high speed impact of fluid along the leading edge of conventional impeller blades. The reduction in shear stress permits the system 100 to handle more sensitive materials which might degrade upon impact with conventional impellers. For instance, the system 100 may provide no to low shear pumping of sensitive materials such as, for example, animal cells. The no to low shear pumping significantly reduces or even eliminates cell damage or death. Further, low to no shear stress of the pumped material may provide higher yields in apheresis, filtration, and other refinement processes.

In an embodiment, the impeller 106 is spaced apart entirely from the housing 104 when the pump 102 is active (i.e., rotationally spinning about axis 126). In another embodiment, the impeller 106 can rest against an inner surface of the housing 104 when the pump is not active. The impeller 106 can have a length, L_{I}, that is less than a length, L_{H}, of the housing 104. In an embodiment, L_{I} is at least 20% L_{H}, at least 30% L_{H}, at least 40% L_{H}, or at least 50% L_{H}. In another embodiment, L_{I} is no greater than 99% L_{H}, no greater than 90% L_{H}, or no greater than 75% L_{H}. The length of the impeller 106 relative to the length of the housing 104 can be in a range between and including any of the values described above.

According to the invention, the pump 102 further includes a magnetic element 128 disposed at least partially in the impeller 106. In an embodiment, the magnetic element 128 can be disposed in any configuration. For instance, the magnetic element 128 can be encased within, embedded in, attached to, or combination thereof to the impeller 106. In an embodiment, the magnet element 128 can be either exposed to the fluid path or protected from the fluid path, depending on the final configuration desired. The magnetic element 128 can include any magnetic, partially magnetic, or ferromagnetic material. The magnetic element 128 only needs to be capable of coupling with a magnetic field supplied by a drive element 130. Accordingly, in a particular embodiment, the magnetic element 128 may be ferromagnetic and selected from the group consisting of a steel, an iron, a cobalt, a nickel, and a rare earth magnet. In an embodiment, the magnetic element 128 is a neodymium rare earth magnet. In a particular embodiment, the neodymium rare earth magnet has desirable power in small configurations. The magnetic element 128 can be configured and chosen depending on the power desired. In a further embodiment, the magnetic element 128 can be selected from any other magnetic or ferromagnetic material as would be readily recognized in the art. In an embodiment, the magnetic element 128 can include a plurality of magnetic elements disposed at least partially, such as fully, within the impeller 106. In a more particular embodiment, the plurality of magnetic elements 128 can be spaced apart from one another, such as spaced apart equal distances from one another. In an embodiment, the magnetic elements 128 can be embedded fully within the impeller 106. This can reduce contact with fluid which might be harmful for particular fluid applications. In another embodiment, the magnetic elements 128 can be at least partially exposed.

As illustrated in FIG. 2, the hub 118 of the impeller 106 can be disposed in a retaining area 132 of the housing 104. The retaining area 132 can form a flanged volume for the previously described fluid bearing by creating an inner surface opposite the hub 118. A ratio of fluid flow rate, F_{B}, through a bearing space 134 to fluid flow rate, F_{A}, through the aperture 116 [F_{B}:F_{A}] is in a range of 1:1000, in a range of 1:100, or in a range of 1:10.

The drive element 130 is adapted to drive, or rotate, the magnetic elements 128 within the impeller 106, thus initiating axial biasing of fluid within the system 100. In an embodiment, the drive element 130 can operate through polarity switching which drives the magnetic elements 128. For instance, the drive element 130 changes polarity thus acting upon each magnet's specific polarity located in the impeller. In another embodiment, the drive element 130 can operate through a rotating magnet mechanism.

In an embodiment, the pump 102 can include one or more stops 136 adapted to prevent over-insertion of the housing 102 into the tubulars 110 and 112. In a particular embodiment, the stops 136 are integral with the housing 102. Further, any flange is envisioned to enable coupling to a range of tubing diameters for the first tubular 110 and the second tubular 112.

In an embodiment, pumps 102 in accordance with one or more embodiments described herein, can be adapted to provide smooth fluid flow within the system 100. That is, unlike some conventional pumps used in fluid systems, the pump 102 can maintain a continuous fluid flow rate differing by no greater than 0.5% at any moment, or no greater than 0.4% at any moment, or no greater than 0.3% at any moment, or no greater than 0.2% at any moment, or no greater than 0.1% at any moment. Such a continuous and unchanging flow rate, coupled with efficiencies and minimum fluid damage exhibited by the impeller 106, can allow for use in certain high precision applications including ocular surgical instrumentation and other delicate areas of operation.

In an embodiment, pumps 102 in accordance with one or more embodiments described herein can be adapted to have a first flow rate, as measured during a first pumping operation (e.g., at time = 0) and a second flow rate, as measured during a second pumping operation (e.g., at time = 45 minutes), where the first and second flow rates differ by no greater than 0.5%, or no greater than 0.4%, or no greater than 0.3%, or no greater than 0.2%, or no greater than 0.1%, and the flow rate is approximately 1 L/minute.

Pumps 102 in accordance with one or more embodiments herein are adapted to be used with aggressive fluids, such as DMSO or phenol. Additionally, pumps 102 in accordance with one or more embodiments described herein can operate at pressure differentials of at least 3 bars, or at least 4 bars, or at least 5 bars.

FIG. 4 includes a schematic view of a system 400 including a first area 402 and a second area 404 spaced apart by a first tubular 406 and a second tubular 408. A pump 410 is disposed between the first and second tubulars 406 and 408. The pump, driven by a drive element 412 is adapted to move fluid from the first area 402 to the second area 404. The pump 410 can have any number of similar characteristics as compared to the pump 102 described above.

Peristaltic pumps are typically used in highly sensitive pumping applications, where finely tuned fluid flow rates are critical. For example, ocular surgery requires highly regulated fluid flow rates to prevent damage to highly fragile ocular tissue. Peristaltic pumps are conventionally utilized in such applications as they deliver highly metered doses of fluid as a result of their design. However, peristaltic pumps delivery highly inconsistent flow rates because of said design. Rollers moving along fluid pathways in the pump can cause erratic pressure gradients and fluid pulsation. Conventional impeller blades also fail to deliver adequate flow rate because of high rotational startup energy and non-uniform flow rates as measured at various locations around the impeller. Further, the damaging nature of the impeller's leading edge can destroy important medium within the fluid. Pumps in accordance with one or more of the embodiments described herein can reduce the problems associated with both peristaltic and conventional impeller-style pumps without compromising performance or fluid delivery. For instance, the wear-free configuration of the pump may eliminate particle shedding (i.e. when two materials generate a frictional force between them) and heat generation to allow for the processing of fluids, cell products, or other materials that are sensitive to the introduction of contaminates, temperature increase, or combination thereof, in the fluid path, to be pumped without the risk of contamination or degradation. For instance, the impeller and housing configuration provides a wear-free pumping action.

Further, given the reusable nature of the drive element 130 and disposable (or single-use) nature of the pump 102, the risk of contamination between successive operations is minimized, unlike with peristaltic pumps, where an entire cassette may need to be disposed after operable use. In an embodiment, the pump 102 can be used in a single limited time (single-use), multiple-use, or continuous use operations. The pump 102 can be used for any industry envisioned that desires no to low shear stress pumping, contaminate-free pumping, consistent fluid flow rates, exposure to an aggressive fluid, or combination thereof. In a particular embodiment, the system 100 may be desirable for particulate-free systems. For instance, the system 100 may be advantageous for the micro-electronics industry where the process fluids used in chip fabrication must remain particulate-free. Industries such as the medical industry, biopharmaceutical industry, pharmaceutical industry, electronic industry, micro-electronic industry, chemical industry, and the like, where pumping is desired is envisioned.

Note that not all of the features described above are required, that a portion of a specific feature may not be required, and that one or more features may be provided in addition to those described. Still further, the order in which features are described is not necessarily the order in which the features are installed.

The following examples are provided to better disclose and teach processes and compositions of the present invention. They are for illustrative purposes only, and it must be acknowledged that minor variations and changes can be made without affecting the scope of the invention as recited in the claims that follow.

### EXAMPLES

### Computer Fluids Dynamics, simulation testing

A CFD is a software used as a simulation technique to understand the behavior of a flow throughout a pump. By simulating the flow throughout a pump, the simulation model presents a complete and clear image of its operation. Conventionally, the CFD model is used identify areas where there is recirculation, flow detachment or where cavitation will occur, and can help establish their causes. The CFD simulation also predicts the flow profiles in the suction pipe or pipes, and can identify any vortex formation which could result in reduced pumping efficiency and premature pump failure.

High-fidelity computational fluid dynamics (Detached Eddy Simulation) and observations during physical testing are used as a mean to demonstrate pump performance and to support achievements and results related to no cavitation, more flow, less torque, and zero to low shear goal.

### Physical Testing

A custom-build pump testing rig is designed with a clear cylindrical housing in order to analyze the flow patterns and cavitation performance. The main components include a cone-bottom hopper tank, recirculation tubing, prototype test section, downstream pressure chamber with 'butterfly' valve, and inverter-controlled motor to belt-drive the rotor pipe.

The test apparatus is equipped with the following sensors: upstream pressure transducer, downstream pressure transducer, paddle wheel flowmeter, thermocouple, optical speed sensors, and wireless rotary torque transducer.

The working fluid in the tests run is ambient temperature tap water. Ambient temperature during test runs is between 66 degrees and 78 degrees Fahrenheit.
Data is logged using Labview systems engineering software.
Cavitation performance is evaluated during design concept phase and results are obtained to successfully achieve no cavitation. The achieved "no cavitation" is verified via high-fidelity computational fluid dynamics (Detached Eddy Simulation) and observations during physical testing (visualization completed in the physical test rig using neutrally-buoyant gel balls). A description of the settings is provided as follows:

### Sample Mesh Details

Total # elements - 110 M
Room - 7.87 M
Inlet Near Field - 2.15M
Inlet - .229 M
Volute - 15.7 M
Housing - 3.75 M
Housing Near Field - 33.5 M
Outlet - 16.2 M
Exit - 30.96 M

The value of "M" is "millions of tetrahedron" as measured by the CFD model; the values showing how many millions of tetrahedron are in different parts of the control volume.

### CFD specifics

Full Unsteady with 0.001, 0.0005 and 0.0002 delta time, 0.2 time up to 1 sec.
Detached Eddy Simulation Turbulence model
RPM 1100, 1650 and 2200
Gravity
Computational Effort - 11 minutes'/time step

Water at nominal ambient temperatures and atmospheric pressure requires pressure of approximately 30,000 - 40,000 Pa to induce a phase change from liquid to liquid vapor. The visualization technique of defining iso-surfaces ("iso" meaning the same value) for negative pressure in the control volume of -20,000Pa or -10,000Pa show that the pump in operation creates a negative pressure, but far less than the required 30,000 - 40,000 Pa needed for cavitation.

Physical tests of volutes are run as noted above. A transparent housing and transparent tubing is used to allow observation at varying RPM and pressure conditions. No bubble formation is seen under any test condition. No cavitation shock/vibration is observed under any test condition.

Observations obtained through physical testing are compared with results obtained in computer visualization, and the downstream flow pattern visualized with gel balls shows the same trends as the computational model. No cavitating flow patterns such as appearance of inchoate bubbles are observed in either of the testing models.

### Flow Rate Performance

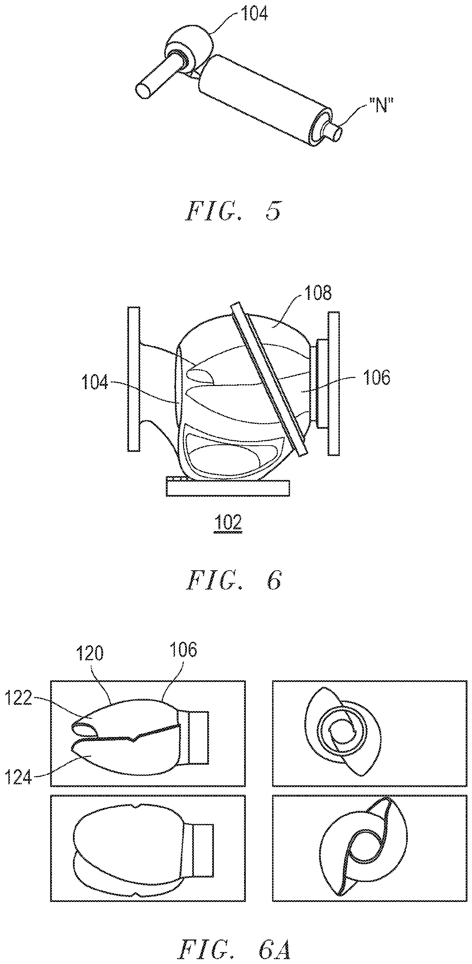

FIG. 5 represents an exemplary geometry for an exemplary housing 104. The housing has a nozzled "exit" cylinder attached. This is a simulation model; the nozzled cylinder is not typically present in a physical experimental rig. The nozzle is analogous to the 'butterfly' valve being adjusted in the experimental rig for the same purpose.

By changing the radius of the exit nozzle on the cylinder (labelled as "N"), the flow rate, pressure drop, and torque of the volute and housing changes. The radius of the nozzle is set to 1.15", 1.0", 0.8", 0.5" and 0.1".

A CFD simulation is then completed for these various radii dimensions and performance values are generated. Refer to Table 1 below.

**Table 1. Comparison of performance parameters**

| | Torque Nm | Mass Flux kg/s | Delta P Pa | |
|---|---|---|---|---|
| | | | | |
| Radius = 1.15" | 4.6 Nm | 12.16 kg/s | 12,000 Pa | |
| Radius = 1.0" | 4.17 Nm | 10.75 kg/s | | |
| Radius = 0.8" | 4.82 Nm | 8.15 kg/s | 35,000 Pa 31,000 Pa | |
| Radius = 0.5" | 3.68 Nm | 3.15 kg/s | 29,000 Pa | |
| Radius = 0.1" | 3.63 Nm | 1375 kg/s | 48,000 Pa | |

Table 1 contains values that demonstrate that the Mass Flow Rate is directly proportional to the Radius of the nozzle. The flow rate increases as the radius of the nozzle is increased.

The fluid flow through the pump may be linear, or divergent through one or more exit paths from greater than 0° to less than 180° on any axis.

### Zero to low shear

The advantageous property of low shear is verified via high-fidelity computational fluid dynamics (Detached Eddy Simulation). CFD simulations directly provide the strain rate terms, which are then multiplied by water's viscosity to give viscous shear stress of the water. Low shear stress is defined to be in the range of 0-200 Pa. The visualization technique of defining iso-surfaces for the pump when operating at 10Pa, 30Pa, and 100Pa generates smaller, and nearly non-existent surfaces respectively for these values of shear stress. 100Pa is still far below the upper limit of 200Pa. Therefore, as defined in scientifically standard terms, the pump demonstrates low shear.

### An Exemplary Pump

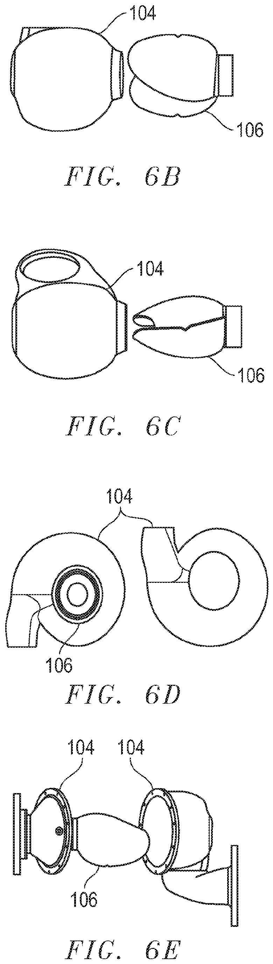

An exemplary pump and housing is designed and can be seen in FIGs. 6, 6a, 6b, 6c, 6d, and 6e. Exemplary design features include, but are not limited to: a rotor scaled to 1" in diameter with universal flanges to attach a range of tubing diameters; a rim drive motor positioned near the inlet with the option of an extended inlet pipe; and a single-use or reusable wet portion of the pump.

FIG. 6 illustrates an exemplary pump 102 including a housing 104 where a portion of the housing is illustrated as semi-transparent to view the impeller 106 contained within the internal volume 108 of the housing. An exemplary impeller is illustrated in FIG. 6a.

FIG. 6a illustrates multiple views of an exemplary impeller 106 with the blade 120 having vanes 122 and 124 with a tulip shape.

FIG. 6b illustrates a side exploded view of an exemplary housing 104 and impeller 106 of FIG. 6a, where the impeller 106 is viewed outside of the internal volume of the housing 104, positioned near the inlet port of the housing 104. The exemplary housing 104 illustrates an embodiment where the inlet port and the outlet port are relatively positioned at a 90 degree angle. FIG. 6c illustrates a view where the housing 104 of FIG. 6b has been rotated to view the outlet of the housing 104.

FIG. 6d includes a top and a bottom view, respectively, of the exemplary housing 104 and impeller 106 of FIGs. 6a and 6b where the impeller is contained within the internal volume of the housing.

FIG.6e includes a top exploded view of an exemplary housing 104 and impeller 106 where the housing is illustrated as two discrete components that are detached and apart.

Any final dimensions and configuration of the housing and impeller are envisioned depending on the final properties and industry desired.

The specification and illustrations of the embodiments described herein are intended to provide a general understanding of the structure of the various embodiments. The specification and illustrations are not intended to serve as an exhaustive and comprehensive description of all of the elements and features of apparatus and systems that use the structures or methods described herein. Separate embodiments may also be provided in combination in a single embodiment, and conversely, various features that are, for brevity, described in the context of a single embodiment, may also be provided separately or in any subcombination. Further, reference to values stated in ranges includes each and every value within that range, including the end range values referenced. Many other embodiments may be apparent to skilled artisans only after reading this specification. Accordingly, the disclosure is to be regarded as illustrative rather than restrictive, the scope of the invention being solely defined by the claims that follow.

## Claims

1. A pump (102) comprising:
a housing (104) defining an internal volume (108);
an impeller (106) disposed in the internal volume (108) of the housing (104), wherein the impeller (106) comprises a magnetic element (128) and at least a portion of the surface of the impeller (106) conforms to a logarithmic spiral,
wherein the impeller (106) comprises a central aperture (116) that extends through the impeller (106) for fluid flow therethrough, wherein the fluid passing around the impeller (106) creates a fluid bearing within the housing (104), and wherein the fluid not directed through the fluid bearing between the housing (104) and impeller (106) passes through the central aperture (116); and
a drive element (130) adapted to magnetically couple with the magnetic element (128) of the impeller (106),
wherein the impeller (106) comprises a hub (118) and a blade (120) extending from the hub (118),
wherein the central aperture (116) extends through the impeller (106), passing through the hub (118) and the blade (120),
wherein the blade (120) includes at least two vanes (122, 124) which have a shape, when viewed along the axis of rotation of the impeller (106), that corresponds to the logarithmic spiral.

2. The pump (102) according to claim 1, wherein the housing (104) defines a curved interior volume (108).

3. The pump (102) according to claim 1, wherein the housing (104) is adapted to couple with a first tubular (110) and a second tubular (112), and wherein the first and second tubulars (110, 112) are associated with a fluid system (100).

4. The pump (102) according to claim 1, wherein the impeller (106) is essentially free of leading edges.

5. The pump (102) according to claim 1, wherein the impeller (106) is adapted to impart a uniform distribution of pressure on the fluid on all surfaces of the impeller (106).

6. The pump (102) according to claim 1, wherein the impeller (106) is adapted to rotate in response to movement by a drive element (130).

7. The pump (102) according to claim 6, wherein the drive element (130) comprises a magnetic drive element, wherein the magnetic drive element is adapted to drive the magnetic element (128) disposed at least partially within the impeller (106), attached to the impeller (106), or combination thereof.

8. The pump (102) according to claim 1, wherein, during use, the pump (102) is adapted to maintain a continuous fluid flow rate differing by no greater than 0.5% at any moment.

9. The pump (102) according to claim 1, wherein the pump (102) has a first flow rate, as measured during a first pumping operation, and a second flow rate during a second pumping operation, and wherein the first and second flow rates differ by no greater than 0.5%.

10. The pump (102) according to claim 1, wherein the pump (102) is adapted to withstand a pressure differential of at least 3 bars or at least 4 bars, or at least 5 bars.

11. The pump (102) according to claim 1, wherein the pump (102) provides a shear stress on a fluid of less than 200 Pascal (Pa).

12. The pump (102) according to claim 1, wherein the pump (102) provides cavitation-free fluid flow.

13. A fluid circulation system (100) comprising:
a first tubular (110);
a second tubular (112); and
a pump (102) according to any one of the preceding claims, the pump (102) being disposed between the first and second tubulars (110, 112) and adapted to urge fluid from the first tubular (110) to the second tubular (112).

## Patentansprüche

1. Pumpe (102), umfassend:
ein Gehäuse (104), das ein Innenvolumen (108) definiert;
ein Laufrad (106), das in dem Innenvolumen (108) des Gehäuses (104) angeordnet ist, wobei das Laufrad (106) ein magnetisches Element (128) umfasst und mindestens ein Abschnitt der Oberfläche des Laufrads (106) mit einer logarithmischen Spirale übereinstimmt,
wobei das Laufrad (106) eine mittlere Öffnung (116) umfasst, die sich durch das Laufrad (106) erstreckt, für einen Fluidfluss dort hindurch, wobei das Fluid, das um das Laufrad (106) herum fließt, ein Fluidlager innerhalb des Gehäuses (104) erzeugt, und wobei das Fluid, das nicht durch das Fluidlager zwischen dem Gehäuse (104) und dem Laufrad (106) geleitet wird, durch die mittlere Öffnung (116) hindurchgeht; und
ein Antriebselement (130), das geeignet ist, um mit dem magnetischen Element (128) des Laufrads (106) magnetisch gekoppelt zu werden,
wobei das Laufrad (106) eine Nabe (118) und ein Schaufelblatt (120), das sich von der Nabe (118) erstreckt, aufweist,
wobei sich die mittlere Öffnung (116) durch das Laufrad (106) erstreckt und durch die Nabe (118) und das Schaufelblatt (120) hindurchgeht,
wobei das Schaufelblatt (120) mindestens zwei Flügel (122, 124) einschließt, die eine Form aufweisen, die, bei Betrachtung entlang der Drehachse des Laufrads (106), der logarithmischen Spirale entspricht.

2. Pumpe (102) nach Anspruch 1, wobei das Gehäuse (104) ein gekrümmtes Innenvolumen (108) definiert.

3. Pumpe (102) nach Anspruch 1, wobei das Gehäuse (104) geeignet ist, um mit einem ersten Rohr (110) und einem zweiten Rohr (112) gekoppelt zu werden, und wobei das erste und das zweite Rohr (110, 112) mit einem Fluidsystem (100) verknüpft sind.

4. Pumpe (102) nach Anspruch 1, wobei das Laufrad (106) im Wesentlichen frei von Vorderkanten ist.

5. Pumpe (102) nach Anspruch 1, wobei das Laufrad (106) geeignet ist, um eine einheitliche Druckverteilung auf das Fluid auf allen Oberflächen des Laufrads (106) auszuüben.

6. Pumpe (102) nach Anspruch 1, wobei das Laufrad (106) geeignet ist, um sich als Reaktion auf eine Bewegung durch ein Antriebselement (130) zu drehen.

7. Pumpe (102) nach Anspruch 6, wobei das Antriebselement (130) ein magnetisches Antriebselement umfasst, wobei das magnetische Antriebselement geeignet ist, um das magnetische Element (128) anzutreiben, das mindestens teilweise innerhalb des Laufrads (106) angeordnet ist, an dem Laufrad (106) angebracht ist oder eine Kombination davon.

8. Pumpe (102) nach Anspruch 1, wobei die Pumpe (102) während des Gebrauchs geeignet ist, um eine kontinuierliche Fluidfließrate aufrechtzuerhalten, die zu keinem Zeitpunkt um mehr als 0,5 % abweicht.

9. Pumpe (102) nach Anspruch 1, wobei die Pumpe (102) eine erste Fließrate, gemessen während eines ersten Pumpvorgangs, und eine zweite Fließrate während eines zweiten Pumpvorgangs aufweist, und wobei die erste und die zweite Fließrate um nicht mehr als 0,5 % voneinander abweichen.

10. Pumpe (102) nach Anspruch 1, wobei die Pumpe (102) geeignet ist, um einem Druckunterschied von mindestens 3 Bar oder mindestens 4 Bar oder mindestens 5 Bar standzuhalten.

11. Pumpe (102) nach Anspruch 1, wobei die Pumpe (102) eine Scherspannung auf ein Fluid von weniger als 200 Pascal (Pa) ausübt.

12. Pumpe (102) nach Anspruch 1, wobei die Pumpe (102) einen kavitationsfreien Fluidfluss bereitstellt.

13. Fluidzirkulationssystem (100), umfassend:
ein erstes Rohr (110);
ein zweites Rohr (112); und
eine Pumpe (102) nach einem der vorstehenden Ansprüche, wobei die Pumpe (102) zwischen dem ersten und dem zweiten Rohr (110, 112) angeordnet ist und geeignet ist, um Fluid von dem ersten Rohr (110) zu dem zweiten Rohr (112) zu treiben.

## Revendications

1. Pompe (102) comprenant :
un boîtier (104) définissant un volume interne (108) ;
une roue (106) disposée dans le volume interne (108) du boîtier (104), dans laquelle la roue (106) comprend un élément magnétique (128) et au moins une partie de la surface de la roue (106) se conforme à une spirale logarithmique,
dans laquelle la roue (106) comprend une ouverture centrale (116) qui s'étend à travers la roue (106) pour permettre l'écoulement de fluide, dans laquelle le fluide passant autour de la roue (106) crée un palier de fluide à l'intérieur du boîtier (104), et dans laquelle le fluide qui n'est pas dirigé à travers le palier de fluide entre le boîtier (104) et la roue (106) passe à travers l'ouverture centrale (116) ; et
un élément d'entraînement (130) adapté pour s'accoupler magnétiquement à l'élément magnétique (128) de la roue (106),
dans laquelle la roue (106) comprend un moyeu (118) et une pale (120) s'étendant à partir du moyeu (118),
dans laquelle l'ouverture centrale (116) s'étend à travers la roue (106), en passant par le moyeu (118) et la pale (120),
dans laquelle la pale (120) comporte au moins deux aubes (122, 124) qui, vues le long de l'axe de rotation de la roue (106), ont une forme qui correspond à la spirale logarithmique.

2. Pompe (102) selon la revendication 1, dans laquelle le boîtier (104) définit un volume intérieur incurvé (108).

3. Pompe (102) selon la revendication 1, dans laquelle le boîtier (104) est adapté pour s'accoupler avec un premier tubulaire (110) et un second tubulaire (112), et dans laquelle le premier et le second tubulaires (110, 112) sont associés à un système de fluide (100).

4. Pompe (102) selon la revendication 1, dans laquelle la roue (106) est essentiellement dépourvue de bords avant.

5. Pompe (102) selon la revendication 1, dans laquelle la roue (106) est adaptée pour donner une distribution uniforme de la pression sur le fluide sur toutes les surfaces de la roue (106).

6. Pompe (102) selon la revendication 1, dans laquelle la roue (106) est adaptée pour tourner en réponse au déplacement d'un élément d'entraînement (130).

7. Pompe (102) selon la revendication 6, dans laquelle l'élément d'entraînement (130) comprend un élément d'entraînement magnétique, dans laquelle l'élément d'entraînement magnétique est adapté pour entraîner l'élément magnétique (128) disposé au moins partiellement dans la roue (106), fixé à la roue (106), ou une combinaison de ceux-ci.

8. Pompe (102) selon la revendication 1, dans laquelle, en cours d'utilisation, la pompe (102) est adaptée pour maintenir un écoulement de fluide continu ne différant pas de plus de 0,5 % à tout moment.

9. Pompe (102) selon la revendication 1, dans laquelle la pompe (102) a un premier écoulement, mesuré pendant une première opération de pompage, et un second écoulement pendant une seconde opération de pompage, et dans laquelle le premier et le second écoulement ne diffèrent pas de plus de 0,5 %.

10. Pompe (102) selon la revendication 1, dans laquelle la pompe (102) est adaptée pour supporter une pression différentielle d'au moins 3 bars ou d'au moins 4 bars, ou d'au moins 5 bars.

11. Pompe (102) selon la revendication 1, dans laquelle la pompe (102) fournit une contrainte de cisaillement sur un fluide de moins de 200 Pascal (Pa).

12. Pompe (102) selon la revendication 1, dans laquelle la pompe (102) fournit un écoulement de fluide sans cavitation.

13. Système de circulation de fluide (100) comprenant :
un premier tube (110) ;
un second tube (112) ; et
une pompe (102) selon l'une quelconque des revendications précédentes, la pompe (102) étant disposée entre le premier et le second tubulaire (110, 112) et adaptée pour pousser le fluide du premier tubulaire (110) vers le second tubulaire (112).
